# EUROPEAN PATENT SPECIFICATION

(11) **EP 2 711 417 B1**
(45) Date of publication and mention of the grant of the patent: **09.11.2016**
(21) Application number: 12006593.3
(22) Date of filing: 20.09.2012
(51) Int. Cl.: B05D 5/00, B32B 3/26, C12N 5/00

(54) **Use of porous hydrophobic polymers filled and coated with water immiscible hydrophobic liquids for protein- and cell-repellent surfaces**
Verwendung poröser hydrophober Polymere, gefüllt und beschichtet mit nicht mit Wasser mischbaren Flüssigkeiten für protein- und zellabstoßende Oberflächen
Utilisation de polymères hydrophobes poreux revêtus et remplis de liquides hydrophobes immiscibles avec l'eau pour surfaces répulsives de protéines et cellules

(43) Date of publication of application: 26.03.2014
(73) Proprietor: Karlsruher Institut für Technologie, 76131 Karlsruhe (DE)
(72) Inventor: Boles, Erica, 76133 Karlsruhe (DE); Levkin, Pavel, Dr., 76344 Eggenstein-Leopoldshafen (DE); Obst, Ursula, Prof. Dr., 76131 Karlsruhe (DE); Schwartz, Thomas, Dr., 76131 Karlsruhe (DE); Li, Junsheng, 76344 Eggenstein-Leopoldshafen (DE)
(74) Representative: Müller-Boré & Partner Patentanwälte PartG mbB

(56) References cited:
- EP-A1- 2 481 794
- WO-A2-2012/100099
- A. K. EPSTEIN ET AL: "From the Cover: Liquid-infused structured surfaces with exceptional anti-biofouling performance", PROCEEDINGS OF THE NATIONAL ACADEMY OF SCIENCES, vol. 109, no. 33, 14 August 2012 (2012-08-14), pages 13182-13187, XP055043847, ISSN: 0027-8424, DOI: 10.1073/pnas.1201973109
- JUNSHENG S. LI ET AL: "Printable Superhydrophilic-Superhydrophobic Micropatterns Based on Supported Lipid Layers", LANGMUIR, vol. 28, no. 22, 5 June 2012 (2012-06-05), pages 8286-8291, XP055043857, ISSN: 0743-7463, DOI: 10.1021/la3010932
- ALBERT R. LIBERSKI ET AL: ""One Cell-One Well": A New Approach to Inkjet Printing Single Cell Microarrays", ACS COMBINATORIAL SCIENCE, vol. 13, no. 2, 14 March 2011 (2011-03-14) , pages 190-195, XP055000736, ISSN: 2156-8952, DOI: 10.1021/co100061c

## Description

The present invention relates to a substrate with a solid support coated with a porous hydrophobic polymer layer or film, wherein said polymer layer or film is coated with a water-immiscible hydrophobic liquid forming a stable film on the polymer surface. The present invention further relates to a method of forming a protein- and cell-repellent surface, comprising the steps of providing a substrate with a solid support coated with a porous hydrophobic polymer layer or film, and applying a water-immiscible hydrophobic liquid to said porous hydrophobic polymer layer or film, a respective method of preventing the formation of biofilms, adhesion of cells and bacteria, and/or migration of cells and bacteria on a surface, as well as the use of a porous hydrophobic polymer layer or film that is coated with a water-immiscible hydrophobic liquid for forming a protein-, cell- and bacteria-repellent surface.

Protein-, cell- and bacteria-repellent surfaces, as well as surfaces preventing cell migration, are of relevance in a wide range of applications including protein, cell, and DNA microarrays, cell patterning, microfluidic devices, biological implants, and non-biofouling surfaces. Further, in many technical, medical, and biotechnological facilities, cell adhesion, e.g. of bacterial cells, leads to the formation of biofilms. These are problematic in many respects, such as hygiene, the clogging of membranes, and an increase of fluid resistance. In particular, bacterial adhesion and biofilm formation represent a complex and serious problem for industry and biomedicine. Over 60% of microbial infections in humans are caused by biofilm-forming bacteria. Biofilms also can cause very serious problems in industrial water systems. While it is important to address the risk caused by biofilms, this is often a difficult task. For example, biofilms can protect their constituent cells in various ways and some cells in biofilms exist in a slow-growing and/or starved state, or adopt a distinct and protected phenotype. Moreover, biocides often cannot penetrate the full depth of the biofilm. The development of a protein- and cell-repellent surface would help to prevent or retard biofilm formation.

The success of respective technologies relies on controlling the adhesion of molecules or cells on surfaces. In particular, cell microarrays require thousands of spatially separated spots of the effectors, such as proteins, small molecules, oligonucleotides, or transfection reagents, so that they only affect the cluster of cells growing on top of each spot and a clear read-out can be obtained. Consequently, large spot-to-spot distances are usually required to separate each spot. The primary limitation of current cell microarrays is that although the effectors are spatially separated, usually the whole surface is seeded with cells and thus the affected and non-affected cells are not physically separated. This also makes it difficult to discern the affected cells within the background of non-affected cells for microscopic analysis. Thus, creating cell microarrays that spatially separate, but also confine, both the effector and the affected cell cluster from one spot to another will significantly ease conducting e.g. high-throughput screens.

Various surface chemistries have already been developed to make non-biofouling surfaces to limit protein and cell adhesion. Polyethylene glycol (PEG) is most commonly used for this purpose, but other coatings such as pluronics F127 (block copolymers based on ethylene oxide and propylene oxide) and hyaluronic acid (HA) are also used. Many different surface chemistry techniques to prevent protein and cell adhesion have been reported. However, many of the surface chemistries used to create non-biofouling surfaces are limited in their method of immobilizing the molecules on the surface. Simply immersing the surface in a chemical solution or stamping the molecule on the surface can result in a non-uniform, low-density, and unstable coating. Micro-contact printing and dip pen nanolithography are also used to print non-biofouling molecules on surfaces, but they require expensive equipment. There are methods to covalently link molecules to surfaces, such as modified alkanethiolate self-assembled monolayers (SAMs), but these modified SAMs can still adsorb significant amounts of serum proteins and they tend to oxidize under ambient conditions, thus restricting their use to short-term experiments. In addition, many cell patterning techniques require serum-free or serum-depleted cell seeding conditions, in addition to surface modification, to limit protein and cell adhesion in the desired areas. This can affect the condition of the cells and possibly the experimental results. Recently, aqueous two-phase systems have been introduced for cell patterning, but improvements in the spatial control and high-throughput possibilities are still needed.

Further, a kind of antimicrobial surface has been developed by incorporating biocides into the surface. This kind of surface exhibits antimicrobial properties when the biocide compounds are released from the surface. Some of these biocide-releasing surfaces have long been used to kill bacteria. However, such biocide-releasing surfaces get exhausted over time.

Furthermore, the development of a cell microarray consisting of a superhydrophilic layer of nanoporous poly(hydroxyethyl methacrylate-co-ethylene dimethacrylate) (HEMA-EDMA) photografted with 2,2,3,3,3-pentafluoropropyl methacrylate (PFPMA) through a quartz chromium photomask to create superhydrophobic regions has recently been reported. When this superhydrophilic-superhydrophobic patterned surface is immersed in an aqueous solution, the regions of nanoporous, superhydrophobic polymer trap air inside the pores and within the asperities of the rough surface, exhibiting a Cassie-Baxter state. These air layers on the surface reduce protein and cell adhesion, and are used to spatially separate and confine clusters of cells in a single spot from the neighboring spots.

In this context, WO 2012/100099 A2 describes self-healing, slippery liquid-infused porous surfaces (SLIPS), as well as methods of making and using the same. Further, Epstein *et al.* (Epstein, A. K. et al.; PNAS, 109(33); 2012; pp. 13182-13187) describes liquid-infused structured surfaces with anti-biofouling properties. Furthermore, EP 2 481 794 A1 describes a method for making arrays or patterns comprising hydrophilic areas and hydrophobic background, as well as related methods of cultivating cells thereon. Moreover, Li *et al.* (Li, J. S. et al.; Langmuir, 28; 2012; pp. 8286-8291) describes printable superhydrophilic-superhydrophobic micropatterns based on supported lipid layers, as well as methods of making the same. Finally, Liberski *et al.* (Liberski, A. R. et al.; ACS Combinatorial Science, 13; 2011; pp. 190-195) describes a method of inkjet printing single cell microassays.

However, there is still room for further improvements concerning the efficiency and stability of protein- and cell-repellent surface.

Accordingly, the technical problem underlying the present invention is to provide an improved protein-, cell- and bacteria-repellent substrate, as well as a simple and rapid method of forming an improved stable protein-, cell- and bacteria-repellent surface, as well as an improved method of preventing the formation of biofilms, adhesion of cells and bacteria and/or migration of cells and bacteria on a surface.

The solution to the above technical problem is achieved by the embodiments characterized in the claims.

In particular, in a first aspect, the present invention relates to a substrate with a solid support coated with a porous hydrophobic polymer layer or film, wherein said polymer layer or film is coated with a water-immiscible hydrophobic liquid forming a stable film on the polymer surface.

In this aspect, the substrate, the solid support, and the porous hydrophobic polymer layer or film are as defined hereinafter. According to the present invention, the porous hydrophobic polymer layer or film comprises a crosslinked polyvinyl monomer and a monovinyl monomer that have been copolymerized in the presence of an inert porogen. Said polyvinyl monomer, monovinyl monomer, and inert porogen can be as defined hereinafter.

The water-immiscible hydrophobic liquid of the substrate of the present invention is selected from the group consisting of perfluorinated lubricants, perfluorinated hydrocarbons, and organosilicone compounds. More preferably, said liquid is a perfluorinated lubricant, selected from the group consisting of perfluoropolyethers, tertiary perfluoroalkylamines, perfluorotri-n-butylamine, perfluoroalkylsulfides, perfluoroalkylsulfoxides, perfluoroalkylethers, perfluorocycloethers, perfluoroalkylphosphines, perfluoroalkylphosphineoxides, long-chain perfluorinated carboxylic acids, fluorinated phosphonic acids, fluorinated silanes, and combinations thereof. Even more preferably, said liquid is a perfluoropolyether, selected from the group consisting of fluorine end-capped hexafluoropropylene epoxide homopolymers, and the KRYTOX^{®} family of lubricants by DuPont^{®}.

According to the present invention, not the entire above porous hydrophobic polymer layer or film is hydrophobic, but the porous hydrophobic polymer layer or film comprises
(i) hydrophilic areas having a desired shape and size; and
(ii) hydrophobic areas having a desired shape and size;
wherein the hydrophilic areas are occupied by an aqueous solution and only the hydrophobic areas are occupied by the water-immiscible hydrophobic liquid. Said aqueous solution is not particularly limited and can be for example water, a cell culture medium, a cell culture medium containing cells, or a solution containing molecules such as e.g. proteins or nucleic acids. According to the present invention, the hydrophilic areas are surrounded by the hydrophobic areas and are separated into a desired spatial pattern. Suitable shapes and sizes of the hydrophilic and hydrophobic areas are not limited and depend only on the particular matter of interest. In a specific embodiment, the substrate used in the method of the present invention is a microarray, e.g. a protein, cell, or DNA microarray.

In this aspect, the hydrophilic areas and the hydrophobic areas can be as defined hereinafter.

In a second aspect, the present invention relates to a method of forming a protein-, cell- and bacteria-repellent surface, comprising the steps of:
(a) providing a substrate with a solid support coated with a porous hydrophobic polymer layer or film; and
(b) applying a water-immiscible hydrophobic liquid to said porous hydrophobic polymer layer or film.

The term "protein-, cell- and bacteria-repellent" as used herein describes the fact that the surfaces formed in the method of the present invention do not allow or do constrain the attachment of any proteins, cells or bacteria. Accordingly, said surfaces do not allow or do constrain the attachment of viruses, prokaryotic cells such as bacteria, eukaryotic cells, or multicellular organisms such as plants or sessile marine organisms.

Means for applying a water-immiscible hydrophobic liquid to said porous hydrophobic polymer layer or film are not particularly limited and include for example the application of said liquid with a syringe or a pipette to the substrate and subsequent washing of the substrate, e.g. with water.

According to the present invention, not the entire above porous hydrophobic polymer layer or film is hydrophobic, but the porous hydrophobic polymer layer or film comprises
(i) hydrophilic areas having a desired shape and size; and
(ii) hydrophobic areas having a desired shape and size;
wherein an aqueous solution is applied to said porous polymer layer or film after step (a) and before step (b), so that said hydrophilic areas are occupied by said aqueous solution and only the hydrophobic areas are occupied by the water-immiscible hydrophobic liquid. In this aspect, the aqueous solution can be as defined above for the substrate of the present invention. According to the present invention, the hydrophilic areas are surrounded by the hydrophobic areas and are separated into a desired spatial pattern. Suitable shapes and sized of the hydrophilic and hydrophobic areas are not limited and depend only on the particular matter of interest. In a specific embodiment, the substrate used in the method of the present invention is a microarray, e.g. a protein, cell, or DNA microarray.

In further embodiments, the surface that is formed in the method of the present invention is a flat surface and does not contain any physical barriers, such as wells, recesses, indentations, depressions, ridges or the like.

Further, in this aspect of the present invention, the water-immiscible hydrophobic liquid, the substrate, the solid support, the porous hydrophobic polymer layer or film, and the hydrophilic and hydrophobic areas are as defined above or hereinafter.

In a third aspect, the present invention relates to a method of preventing or retarding the formation of biofilms, adhesion of cells and bacteria, and/or migration of cells and bacteria on a surface or on parts of a surface, comprising the steps of:
(a) providing on said surface or parts of a surface a porous hydrophobic polymer layer or film; and
(b) applying a water-immiscible hydrophobic liquid to said porous hydrophobic polymer layer or film.

The term "biofilm" as used herein relates to any aggregate of cells in which the cells adhere to each other on a surface. Cells that form a biofilm may be prokaryotic or eukaryotic cells or viruses.

The surface on which prevention or retardation of the formation of biofilms is intended is not particularly limited, provided that a porous hydrophobic polymer layer or film in accordance with the present invention can be coated on said surface.

Further, in this aspect of the present invention, the water-immiscible hydrophobic liquid, means for applying the same to said porous hydrophobic polymer layer or film, as well as said porous hydrophobic polymer layer or film are as defined above or hereinafter.

In a fourth aspect, the present invention relates to the use of a porous hydrophobic polymer layer or film that is coated with a water-immiscible hydrophobic liquid for forming a protein-, cell- and bacteria-repellent surface.

In this aspect, the water-immiscible hydrophobic liquid, the surface, means for applying the same to said porous hydrophobic polymer layer or film, as well as said porous hydrophobic polymer layer or film, are as defined above or hereinafter.

Further, described herein is a method for forming a cell array, said method comprising the steps of:
(a) providing a substrate according to the present invention, wherein the porous hydrophobic polymer layer or film comprises hydrophilic areas having a desired shape and size and hydrophobic areas having a desired shape and size as defined above;
(b) applying an aqueous solution containing the cells to the porous polymer layer or film; and
(c) applying a water-immiscible hydrophobic liquid to said porous polymer layer or film.

The term "cell array" as used herein refers to a substrate comprising a multitude of separated and confined areas wherein cells can be kept in an aqueous medium. According to the present invention, said cells cannot grow on or migrate across the hydrophobic areas being coated with a water-immiscible hydrophobic liquid. The term "cell" as used in this context is not particularly limited and relates to any prokaryotic or eukaryotic cells.

In this aspect, the substrate, the solid support, the porous hydrophobic polymer layer or film, the hydrophilic and hydrophobic areas, and the water-immiscible hydrophobic liquid are as defined above or hereinafter.

Further, described herein is the use of a porous hydrophobic polymer layer or film that is coated with a water-immiscible hydrophobic liquid for preventing the migration of cells across said porous hydrophobic polymer layer or film.

In this aspect, the substrate, the solid support, the porous hydrophobic polymer layer or film, the hydrophilic and hydrophobic areas, and the water-immiscible hydrophobic liquid are as defined above or hereinafter.

In all aspects of the present invention, the substrates used, methods for producing the same, the porous polymer layers or films used, methods for producing the same, as well as methods of applying these to a surface, are preferably as described hereinafter.

In the following, the terms "hydrophilic" and "superwetting" will be used synonymously.

In general, surface properties can be classified into hydrophobic and hydrophilic surfaces depending on the value of the water contact angle (WCA). A surface having a WCA greater than 90° is referred to as hydrophobic, whereas a WCA smaller than 90° is referred to as hydrophilic. The maximum water contact angle on a smooth surface is about 120°. In practice, two types of WCA values are used: static and dynamic. Static water contact angles (qstat) are obtained by sessile drop measurements, where a drop is deposited on the surface and the value is obtained by a goniometer or a specialized software. Dynamic contact angles are non-equilibrium contact angles and are measured during the growth (advancing WCA qadv) and shrinkage (receding WCA qrec) of a water droplet. The difference between qadv and qrec is defined as contact angle hysteresis (CAH). In a preferred embodiment of the present invention, surfaces with high WCA, preferably greater than 130°, more preferably greater than 140°, most preferably greater than 150°, and/or low water CAH (less than about 30°, preferably less than about 20°, more preferably less than about 10°) are called superhydrophobic. Water droplets do not stick to such surfaces and simply roll off. In a preferred embodiment of the present invention, surfaces with both static, advancing and receding WCAs less than 20°, preferably less than 10°, more preferably less than 5°, most preferably close to or of 0° are called superhydrophilic. Water droplets are rapidly absorbed by such surfaces.

In a preferred embodiment of the present invention, the substrate used is a surface having hydrophilic, preferably superhydrophilic, patterns on a hydrophobic, preferably superhydrophobic, background. In a more preferred embodiment of the present invention, the patterned substrate is a surface having 6, 12, 24, 96, 384, 1536 hydrophilic, preferably superhydrophilic, areas on a hydrophobic, preferably superhydrophobic, background.

Superhydrophobic and superhydrophilic materials are known in the art. Within the scope of the present invention, the porous polymer layer which exhibits hydrophobic, preferably superhydrophobic, properties is comprised of a crosslinked polyvinyl monomer, wherein the polyvinyl monomer can be one or more monomers selected from the group consisting of alkylene diacrylates, oligoethyleneoxide diacrylates or dimethacrylates, alkylene dimethacrylates, pentaerythritol tetraacrylate, pentaerythritol tetramethacrylate, trimethylopropane acrylate, trimethylopropane methacrylate, divinylbenzene, and divinylnaphthalene. In a preferred embodiment, the polyvinyl monomer is selected from the group consisting of ethylene dimethacrylate and divinylbenzene, whereof ethylene dimethacrylate is particularly preferred.

The hydrophobic, preferably superhydrophobic, porous polymer may further comprise a monovinyl monomer, wherein the monovinyl monomer can be selected from the group consisting of alkyl acrylates, alkyl methacrylates, aryl acrylates, aryl methacrylates, aryl alkyl acrylates, aryl alkyl methacrylates, fluorinated alkyl acrylates, fluorinated alkyl methacrylates, styrene, vinylnaphthalene, vinylanthracene, and derivatives thereof, wherein the alkyl group in each of the alkyl monomers has 1-18 carbon atoms. In a preferred embodiment, the monovinyl monomer is selected from the group consisting of methyl methacrylate, butyl methacrylate, benzyl methacrylate and styrene. The above polyvinyl monomer is copolymerized with the monovinyl monomer affording the porous hydrophobic, preferably superhydrophobic, polymer layer.

In a particularly preferred embodiment of the present invention, the porous polymer layer comprises at least one member selected from the group consisting of porous poly(butyl methacrylate-co-ethylene dimethacrylate) (BMAcoEDMA), porous poly(methyl methacrylate-co-ethylene dimethacrylate) (MMAcoEDMA), porous poly(2-hydroxyethyl methacrylate-co-ethylene dimethyl-acrylate) (HEMAcoEDMA), porous poly(styrene-co-1,4-divinylbenzene) (STcoDVB), porous poly(2,2,3,3,3-pentafluoropropyl methacrylate-co-ethylene dimethacrylate) (PFPMAcoEDMA).

In a preferred embodiment of the substrate used in the present invention, the pattern is created by modifying the surface of the porous polymer layer using photografting through a photomask. In a more preferred embodiment of the present invention, the hydrophilic areas are created by photografting the porous polymer layer being a porous hydrophobic layer.

In a preferred embodiment of the present invention, the substrate is a microarray which preferably exhibits an array-pattern which is created by photografting the porous polymer layer using a photomask. The use of photochemical methods allows precisely controlling the geometry, size and distance between the spots by the design of the photomask.

According to the present invention, the porous polymer layer itself is hydrophobic, preferably superhydrophobic. In this embodiment, the hydrophobic, preferably superhydrophobic, areas originate from the porous polymer layer, whereas the hydrophilic, preferably superhydrophilic, spots/areas are created by modifying, preferably photografting the porous polymer layer.

According to the present invention, the hydrophilic, preferably superhydrophilic, areas may be created by modifying, preferably photografting the porous polymer layer being a porous hydrophobic, preferably superhydrophobic, polymer layer. In a preferred embodiment of the present invention, imparting hydrophilic, preferably superhydrophilic, properties to the hydrophobic, preferably superhydrophobic, porous polymer layer is accomplished using a hydrophilic compound, for example mono- or polyvinyl monomer selected from the group consisting of monovinyl monomer, wherein the monovinyl monomer is selected from the group consisting of vinylacetate, vinylpyrrolidone, acrylic acid, methacrylic acid, methacrylamide, acrylamide, alkyl derivatives of methacrylamide, alkyl derivatives of acrylamide, wherein the alkylene group consists of 1-4 carbon atoms, hydroxyalkyl acrylates and acrylamides, hydroxyalkyl methacrylates and methacrylamides, oligoethylene glycol acrylates and oligoethylene glycol methacrylates, potassium 3-sulfopropyl acrylate, potassium 3-sulfopropyl methacrylate, 2-acryloamido-2-methyl-1-propanesulfonic acid, 2-acrylamido-glycolic acid, [2-(methacryloyloxy)ethyl] trimethylammonium chloride, N-[3-(dimethylamino)propyl] methacrylamide or alkylene diacrylates, oligoethyleneoxide diacrylates or dimethacrylates, alkylene dimethacrylates, pentaerythritol tetraacrylate, pentaerythritol tetramethacrylate. Preferably, said hydrophilic compound is a photografting mixture comprising [2-(methacryloyloxy)ethyl]-trimethylammonium chloride (META) or 2-acryloamido-2-methyl-1-propanesulfonic acid. According to a more preferred embodiment, the hydrophilic photografting mixture consists of 15wt.% META, and 0.25wt.% benzophenone dissolved in a 1:3 (v/v) mixture of water and tert-butanol.

In the thus obtained pattern, an easy and homogeneous adsorption of the spotting solution in the porous hydrophilic areas can be guaranteed. Each area is separated from adjacent areas with hydrophobic, preferably superhydrophobic, areas that render the areas impermeable for all water based solutions.

In a preferred embodiment of the present invention, the hydrophilic areas of the substrate are separated by the hydrophobic areas so as to form an array of hydrophilic areas surrounded by a hydrophobic porous polymer surface.

In another preferred embodiment of substrate according to the present invention, the hydrophilic areas are separated by the hydrophobic, preferably superhydrophobic, areas of porous polymer surface so as to form hydrophilic, preferably superhydrophilic, channels. In a more preferred embodiment of the present invention, the substrate comprises hydrophilic, preferably superhydrophilic, channels on a hydrophobic, preferably superhydrophobic, background and the substrate is used for microfluidic applications.

In another preferred embodiment of the present invention, the hydrophilic, preferably superhydrophilic, areas which are separated by the hydrophobic, preferably superhydrophobic, areas in the substrate have a square shape. Hydrophilic, preferably superhydrophilic, areas having a square shape allow dense packing of the areas and ease the readout. Nevertheless, the substrate according to the present invention is not limited to any array-format, but represents a more general and novel approach that allows patterning and for example culturing of cells in a predesigned spatial order.

In one embodiment of the present invention, the area-density of the hydrophilic, preferably superhydrophilic, areas is increased. Preferably, the amount of hydrophilic, preferably superhydrophilic, areas is at least 200 per cm² of the patterned substrate, more preferably at least 300 per cm² of the patterned substrate, even more preferably at least 400 per cm² of the patterned substrate. In a particularly preferred embodiment of the present invention, the amount of hydrophilic, preferably superhydrophilic, areas is at least 500 per cm² of the patterned substrate.

According to an even more preferred embodiment of the present invention, the substrate used in the present invention has a size which fits on a standard microtiter scaled plate (12 x 8 cm), preferably a size of about 11 x 7 cm with at least 40000, preferably at least 50000 hydrophilic areas separated by hydrophobic, preferably superhydrophobic, background. In another preferred embodiment of the present invention, the substrate used in the present invention is a microslide of about 1 x 7 cm.

Preferably, in the substrate used in the present invention, the overall area-number on a single chip is significantly increased. When used as a cell microarray, minimal area of a single area in the patterned substrate according to the present invention is limited by the amount of cells needed in each area to obtain statistically valid data. In a particularly preferred embodiment of the present invention, each of the hydrophilic, preferably superhydrophilic, areas defined in (i) has a side length of 1000 µm or less, preferably 600 µm or less, more preferably 400 µm or less, and most preferably 335 µm or less, particularly under the provision that the hydrophilic, preferably superhydrophilic, areas have a square shape. In another preferred embodiment of the present invention, the size of the hydrophilic, preferably superhydrophilic, areas is large enough to accommodate at least 100 cells, preferably 200 cells, more preferably at least 300 cells.

In a preferred embodiment of the present application the hydrophobic, preferably superhydrophobic, areas have a width of 500 µm or less, preferably 200 µm or less, more preferably 100 µm or less and even more preferably 60 µm or less.

Further, according to the present invention, the thickness of the porous polymer layer is not limited. Regarding transparency properties or reducing manufacturing costs, a thin porous polymer layer is preferred. Particularly preferred is a porous polymer layer having a thickness of 45 µm or less, preferably 30 µm or less, and more preferably 15 µm or less.

The substrate used in the present can comprise at least one biologically active agent provided in at least one hydrophilic, preferably superhydrophilic, area. According to a preferred embodiment, each hydrophilic, preferably superhydrophilic, area of the patterned substrate is provided with at least one biologically active agent.

The substrate used in the present invention can be manufactured by a method, comprising the steps of:
(a) providing a polymerization mixture between two solid supports;
(b) polymerizing the polymerization mixture to form a porous polymer layer;
(c) removing one of the two solid supports; and
(d) modifying the surface of the porous polymer layer, thereby creating a pattern of hydrophilic areas surrounded by hydrophobic areas.

The solid support may be of any material known in the art which is solid, preferably at a temperature within the range of -30°C to 130°C, more preferably within the range of 0°C to 40°C, more preferably at room temperature. The solid support can be selected from the group consisting of glass, metal, such as a stainless steel plate, or an aluminum foil, plastic, concrete, wood, and masonry. Preferably, the solid support is transparent, wherein a glass solid support is particularly preferred. Such a glass solid support may be activated and/or modified prior to use.

In order to achieve covalent attachment of the porous polymer layer, the glass surface is preferably first activated, and then functionalized with an anchor-group. For example, cleaned glass plates may be immersed in 1 M NaOH for one hour and afterwards washed with deionized water followed by immersing them in 1 M HCl for 30 min, washing with deionized water and drying with a nitrogen gun.

Further, the glass solid support may be modified by for example dropping several drops of a solution containing 20% 3-(trimethoxysilyl)propyl methacrylate in ethanol (adjusted to pH = 5 with acetic acid) on an activated glass plate, covering the glass plate with another activated glass plate, thereby ensuring that no bubbles are trapped between the two glass plates, reapplying said solution after 30 minutes and after another 30 minutes washing the glass plates with acetone and drying with a nitrogen gun.

The glass solid supports may be made inert for example by fluorination using for example tridecafluor-(1,1,2,2)-tetrahydrooctyltrichlorosilane placed in a vacuumed desiccator together with the glass solid support for 2h.

In step (a) of the method for the manufacture of a substrate to be used in the method of the present invention, a polymerization mixture is provided between two solid supports. The solid supports may be made of the same material, or of different materials. In particular, the aforementioned materials suitable for the substrate according to the present invention are preferred. Preferably, the solid supports are made of a glass material, of which one or both may be modified and/or activated prior to use, such as a glass modified with anchor groups. Preferably, the solid support is a solid support as defined herein.

In step (b) of the method for the manufacture of a substrate, polymerization is carried out in the mold between the two solid supports. The polymerization may involve a free radical polymerization of a polyvinyl monomer and a monovinyl monomer in the presence of porogenic solvents. The free radical initiation is preferably done either thermally or by irradiation with UV- or visible light or γ-irradiation. When polymerization is carried out by thermal initiation, the thermal initiator is generally a peroxide, a hydroperoxide, or an azo-compound selected from the group consisting of benzoyl peroxide, potassium peroxodisulfate, ammonium peroxodisulfate, t-butyl hydroperoxide, 2,2'-azobisiobutyronitrile (AIBN), and azobisisocyanobutyric acid, and the thermally induced polymerization is performed by heating the polymerization mixture to temperatures between 30°C and 120°C. Polymerization can also be achieved using photoinitiators including, but not limited to, benzophenone, 2,2-dimethoxy-2-phenylaceto-phenone, dimethoxyacetophenone, xanthone, thio-xanthone, camphorquinone their derivatives, and mixtures thereof.

The polymerizing mixture is not limited, as long as said polymerizing mixture affords a porous polymer layer having hydrophobic, preferably superhydrophobic, properties. Depending on the aimed surface property of the porous polymer, i.e. hydrophobicity, the respective monomers are selected accordingly.

The porogen (i.e. liquids that cause formation of the porous structure of the polymer) used to prepare the porous polymer layer may be selected from a variety of different types of compounds. For example, suitable liquid porogens include aliphatic hydro-carbons, aromatic hydrocarbons, esters, amides, alcohols, ketones, ethers, solutions of soluble polymers, and mixtures thereof. For preparation of hydrophilic, preferably superhydrophilic, materials, water may also be used. The porogen is generally present in the polymerization mixture in an amount of from about 40 to 90 vol%, more preferably from about 50 to 80 vol%. In a preferred embodiment, the porogen is a mixture of 1-decanol and cyclohexanol.

Preferably, the polyvinyl crosslinker is selected from the group consisting of alkylene diacrylates, alkylene dimethacrylates, alkylene diacrylamides, alkylene dimethacrylamides, hydroxyalkylene diacrylates, hydroxyalkylene dimethacrylates, wherein the alkylene group consists of 1 - 4 carbon atoms, oligoethylene glycol diacrylates, vinyl esthers of polycarboxylic acids, pentaerythritol diacrylate, pentaerythritol triacrylate, pentaerythritol dimethacrylate, and pentaerythritol trimethacrylate.

Preferably, the monovinyl monomers are selected from the group consisting of alkyl vinylacetate, vinylpyrrolidone, acrylic acid, methacrylic acid, methacrylamide, acrylamide, alkyl derivatives of methacrylamide, alkyl derivatives of acrylamide, wherein the alkylene group consists of 1 - 4 carbon atoms, hydroxyalkyl acrylates and acrylamides, hydroxymethacrylates and methacrylamides, oligoethylene glycol acrylates and oligoethylene glycol methacrylates, potassium 3-sulfopropyl acrylate, potassium 3-sulfopropyl methacrylate, 2-acryloamido-2-methyl-1-propanesulfonic acid, 2-acrylamidoglycolic acid, [2-(methacrylooxy)ethyl] trimethylammonium chloride, and N-[3-(dimethylamino)propyl]methacrylamide.

Preferably, the inert porogen is selected from the group consisting of aliphatic hydrocarbons, aromatic hydrocarbons, esters, amides, alcohols, ketones, ethers, solutions of soluble polymer, water, and mixtures thereof.

Preferably, the porous polymer layer comprises at least one member selected from the group consisting of porous poly(butyl methacrylate-co-ethylene dimethacrylate) (BMAcoEDMA), porous poly(methyl methacrylate-co-ethylene dimethacrylate) (MMAcoEDMA), porous poly(2-hydroxyethyl methacrylate-co-ethylene dimethyl-acrylate) (HEMAcoEDMA), porous poly(styrene-co-1,4-divinylbenzene) (STcoDVB), porous poly(2,2,3,3,3-pentafluoropropyl methacrylate-co-ethylene dimethacrylate) (PFPMAcoEDMA).

More preferably, the hydrophilic porous polymer layer is made of poly(2-hydroxyethyl methacrylate-co-ethylene dimethacrylate) synthesized by copolymerization of 2-hydroxyethyl methacrylate (HEMA) and ethylene dimethacrylate (EDMA) in the presence of.

Preferably, the porous polymer layer is made of poly(butyl methacrylate-co-ethylene dimethyl-acrylate) (BMAcoEDMA). The porous polymer layer of BMAcoEDMA is synthesized by copolymerization of butyl methacrylate (BMA) and ethylene dimethacrylate (EDMA) in the presence of porogens.

In the embodiment that the porous polymer layer exhibits hydrophobic, preferably superhydrophobic, properties, the polyvinyl monomer is one or more monomers selected from the group consisting of alkylene diacrylates, alkylene dimethacrylates, penta-erythritol tetraacrylate, pentaerythritol tetramethacrylate, trimethylopropane acrylate, trimethylopropane methacrylate, divinylbenzene, and divinyl-naphthalene. Preferably, the polyvinyl monomer is selected from the group consisting of ethylene dimethacrylate and divinylbenzene, whereof ethylene dimethacrylate is particularly preferred.

Further, the hydrophobic, preferably superhydrophobic, porous polymer further comprises a monovinyl monomer, wherein the monovinyl monomer is selected from the group consisting of alkyl acrylates, alkyl methacrylates, aryl acrylates, aryl methacrylates, aryl alkyl acrylates, aryl alkyl methacrylates, fluorinated alkyl acrylates, fluorinated alkyl methacrylates, styrene, vinylnaphthalene, vinylanthracene, and derivatives thereof, wherein the alkyl group in each of the alkyl monomers has 1-18 carbon atoms. Preferably, the monovinyl monomer is selected from the group consisting of methyl methacrylate, butyl methacrylate, benzyl methacrylate and styrene. The above polyvinyl monomer is copolymerized with the monovinyl monomer in the presence of a porogenic solvent affording the porous hydrophobic, preferably superhydrophobic, polymer layer.

The ratio of polyvinyl monomer to monovinyl monomer is not limited as long as the obtained porous polymer exhibits hydrophobic, preferably superhydrophobic, properties. In a preferred embodiment, the polyvinyl monomer content is within the range of 5 to 30wt.%, preferably 10 to 25wt.%, more preferably 15 to 20wt.%, based on the total amount of the polymerization mixture. The monovinyl monomer content is preferably within the range of 10 to 50wt.%, preferably 15 to 45wt.%, more preferably 20 to 40wt.%, based on the total amount of the polymerization mixture.

Particularly preferred polymerization mixtures include:
(i) poly(2-hydroxyethyl methacrylate-co-ethylene dimethacrylate) (HEMA-co-EDMA): 2-hydroxyethyl methacrylate (HEMA) (24wt.%), ethylene dimethacrylate (EDMA) (16wt.%), 1-decanol (12wt.%), Cyclohexanol (48wt.%) and 2,2-dimethoxy-2-phenylaceto-phenone (DMPAP) (1wt.% with respect to monomers),
(ii) poly(2-hydroxyethyl methacrylate-co-ethylene dimethacrylate): 2-hydroxy-ethyl methacrylate (24wt.%), ethylene dimethacrylate (16wt.%), 1-decanol (40wt.%), cyclohexanol (20wt.%) and 2,2-dimethoxy-2-phenylacetophenone (1wt.% with respect to monomers), and
(iii) poly(butyl methacrylate-co-ethylene dimethacrylate) (BMAcoEDMA): butyl meth-acrylate (BMA) (24wt.%), ethylene dimethacrylate (16wt.%), 1-decanol (40wt.%), cyclohexanol (20wt.%) and 2,2-dimethoxy-2-phenylacetophenone (1wt.% with respect to monomers).

After completion of the polymerization, one of the two substrates is removed in step (c).

Preferably, the modification of the surface of the porous layer in step (d) of the method for the manufacture of a substrate is carried out by photografting the surface of the porous polymer layer using a photomask, wherein a photografting mixture is provided on the surface of the porous polymer layer. As described earlier, the photomask may be applied so as to create the pattern, or to create the areas. Depending on the nature of the porous polymer layer (hydrophobic, preferably superhydrophobic), the photografting mixture applied to the porous polymer layer induces hydrophilic, preferably superhydrophilic, regions on the porous polymer layer.

The preparation of a substrate ready for further applications such as printing biomolecules takes preferably about one hour.

The method for the manufacture of a patterned substrate described herein preferably further comprises the step (e) providing at least one biologically active agent in at least one hydrophilic, preferably superhydrophilic, area, more preferably in each of the hydrophilic, preferably superhydrophilic, areas.

In particular embodiments, the substrates used, methods for producing the same, the porous polymer layers or films used, methods for producing the same, as well as methods of applying these to a surface, are as described in European patent application 10 015 106.7 and/or as described in Geyer, F. L. et al., Superhydrophobic-Superhydrophilic Micropatterning: Towards Genome-on-a-Chip Cell Microarrays, Angewandte Chemie International Edition 2011, 50, pp. 8424-8427.

The present invention solves the problem of creating stable, protein- and cell-repellent surfaces, as well as surfaces preventing cell migration, by coating a porous, hydrophobic surface with a layer of a water-immiscible hydrophobic liquid. This creates a hydrophobic, liquid-like surface which proteins do not readily adhere to and, thus, adherent cells cannot grow, attach or migrate on this surface. The water-immiscible hydrophobic liquid layer penetrates the highly porous hydrophobic polymer layer or film and does not easily wash away and thus remains stable. In addition to rendering whole surfaces protein- and cell-repellent, porous hydrophilic-hydrophobic patterned surfaces can be used to pattern the water-immiscible hydrophobic liquid on the hydrophobic regions surrounded by hydrophilic areas coated with an aqueous solution and thus selectively create cell-repellent and migration-resistant areas. This invention eliminates the need for physical barriers to separate cells in different spots, such as when using microtiter plates, and instead uses a liquid barrier. It is useful for applications such as cell or bacteria microarrays, cell or bacteria patterning, anti-fouling surfaces, and antimicrobial applications.

More specifically, the chemistry and properties of porous, hydrophobic polymers has been modified with a water-immiscible hydrophobic liquid to render them oleophobic, non-biofouling, and still hydrophobic. In addition, these coatings are stable and self-repairing. These surface characteristics are advantageous for applications which do not want non-specific protein or cell adhesion on surfaces, such as in cell patterning, cell/protein/DNA microarrays, and biomedical surfaces. This invention is different from other methods, such as surface modification with PEG, because it uses a water-immiscible hydrophobic liquid as the non-biofouling component. It is also a simple and rapid method.

In conclusion, a method to modify the chemistry and properties of a porous, hydrophobic polymer with a hydrophobic liquid to render it protein- and cell-repellent and stable for cell culture is provided. In addition to rendering whole surfaces protein- and cell-repellent, porous hydrophilic-hydrophobic patterned surfaces can be used to fill only the hydrophobic regions with the hydrophobic liquid, thus selectively creating protein and cell-repellent areas. This is useful e.g. for the stringent confinement of transfection mixtures and cells to localized and addressable locations for cell microarrays. The hydrophobic liquid barriers allow increased spot density, confine solutions in one spot, prevent cell migration between the neighboring spots, and reduce cell adhesion on the barriers without the use of serum-free medium. In addition, respective arrays are compatible with high-throughput screening methods because of the sample transparency for inverted microscopy and the standard glass slide size which is compatible with slide holders for microscopy and microarray scanners. It is envisioned that such arrays will be a useful tool for many non-biofouling applications.

The figures show:
Figure 1:
   Schematic of the formation of a hydrophobic, liquid-like pattern.
      a) A superhydrophilic, nanoporous polymer surface is photografted with superhydrophobic moieties. When the superhydrophilic-superhydrophobic patterned substrate is immersed in water, the superhydrophilic areas become easily wetted while the superhydrophobic areas remain in a dry, Cassie-Baxter state. When the substrate is pulled out of water, only the superhydrophilic areas remain filled with water and distinct droplets are formed to obtain homogeneous arrays of droplets. b) A thin layer of hydrophobic liquid is applied over the array of droplets. The surface is rinsed with water to wash off the unstable hydrophobic liquid layer covering the water droplets. c) Water droplets formed in the superhydrophilic areas are separated by the hydrophobic liquid barriers. d) Cells adhere to the superhydrophilic areas separated by the hydrophobic liquid barriers.
Figure 2:
   Water droplets in superhydrophilic spots separated by Krytox barriers for different array pattern geometries.
      a) 1 mm side length square, 100 µm barrier. b) 1 mm diameter circle, 100 µm barrier. c) 1 mm side length triangle, 100 µm barrier.
Figure 3:
   Different LiquidArray pattern geometries cultured with cells for 3 days. Bottom row/panel shows fluorescent intensity profile plots for the ROI indicated in each image. a) HT1080-eGFP cells seeded at 30,000 cell/cm² on 1 mm side length hexagon, 100 µm barrier LiquidArray. b) HEK 293 cells seeded at 30,000 cell/cm² on 500 mm side length square, 100 µm barrier LiquidArray. DAPI-stained. c) MTIy-CMV-eGFP cells seeded at 60,000 cell/cm² on 1 mm side length square, 100 µm barrier LiquidArray.
Figure 4:
   Influence of LiquidArray pattern geometry on cell-repellency.
      HT1080-eGFP cells seeded at 30,000 cell/cm² and cultured for 2 days. a) 500 mm side length square, 100 µm barrier LiquidArray. b) 1 mm side length hexagon, 100 µm barrier LiquidArray.
Figure 5:
   Comparison between Cassie-Baxter air and hydrophobic liquid barriers.
      HEK 293 cells seeded at 30,000 cell/cm² in a single superhydrophilic square of 3 mm side length, 100 µm barrier and cultured for 3 days. DAPI-stained. a) Cassie-Baxter air barrier, day 1. b) Cassie-Baxter air barrier, day 3. c) Hydrophobic liquid barrier, day 1. d) Hydrophobic liquid barrier, day 3. e) Box plot of the area fraction of cells in the superhydrophilic square (SL) vs. the Cassie-Baxter (CB) air barriers or the hydrophobic liquid (Krytox) barriers at day 1 and 3.
Figure 6:
   Cell viability tests on Cassie-Baxter and LiquidArray patterns (500 µm square, 100 µm barrier).
      HEK 293 cells seeded at 30,000 cell/cm² and cultured for 3 days, and then stained with Calcein AM and propidium iodide. a) Cassie-Baxter air barriers. b) Hydrophobic liquid barriers.
Figure 7:
   HEK 293 cells reversely transfected on LiquidArrays.
      a) Plasmid DNA pCS2+-GFP (upper right panel) and pCS2+-mCherry (lower right panel) were pre-printed in the superhydrophilic spots of the LiquidArray pattern. HEK 293 cells were seeded at 30,000 cell/cm² and cultured for 3 days, and then fixed and stained with Hoechst 33342.
   Figure 8:
      Water contact angle changes of the uncoated porous surface and lubricant coated surface after keeping in air and in a CDC biofilm reactor under shear force after 7 days.
Figure 9:
   (A) Fluorescence images of a reference glass slide and a lubricant coated surface stained with CTC (light grey) and DAPI (dark grey) after a 7-day incubation (in a biofilm Plug Flow Reactor, flow rate: 0.94 mL min-1) with *P. aeruginosa* cultivated in BM2 minimal medium. (B) Fluorescence images of a scratched lubricant coated surface stained with CTC (light grey) and DAPI (dark grey) before (left) and after (right) a 14-day incubation (in a biofilm Plug Flow Reactor, flow rate: 0.94 mL min-1) with *P. aeruginosa* cultivated in BM2 minimal medium.

The present invention will now be further illustrated in the following examples without being limited thereto.

### Examples

### Experimental procedures:

### General.

All polymerizations and photografting were carried out on an OAI Model 30 deep-UV collimated light source (San Jose, CA, USA) fitted with an USHIO 500 W Hg-xenon lamp (Japan). Irradiation intensity was calibrated to 12 mW/cm² (5.10 mW/cm² after cover glass slide, 4.77 mW/cm² after cover glass slide and photomask) using an OAI. 360 UV power meter with a 260 nm probe head. Schott (Germany) Nexterion Glass B UV transparent glass plates were used as substrates for polymer layers. Monomers were purchased from Sigma-Aldrich. Biochemicals were purchased from Invitrogen.

### Array preparation - Glass Surface Modification.

To achieve covalent attachment of the polymer layer, the glass surfaces are first activated and then functionalized with an anchor group for methacrylates. Activation of glass slides: Clean glass slides are immersed in 1 M NaOH for 1 h and afterwards washed with deionized water, and then immersed in 1 M HCl for 30 min, washed with deionized water, and dried with a nitrogen gun. Modification of glass slides: Several drops of a solution containing 20 %vol. 3-(trimethoxysilyl)propyl methacrylate in ethanol, adjusted to pH 5 with acetic acid, are dropped on an activated glass slide. The plate is covered with another activated glass slide. No bubbles should be trapped between the two slides. The solution is reapplied after 30 min. After another 30 min, the slides are washed with acetone and dried with a nitrogen gun.

Fluorination of glass slides: An activated glass slide is placed in a vacuumed desiccator together with a vial containing several drops of tridecafluoro-(1,1,2,2)-tetrahydrooctyltrichlorosilane over night.

### Array preparation - Polymerization Procedure and Photografting.

Polymerization: Two strips of Teflon film (American Durafilm Co.), defining the thickness of the polymer layer, are placed at the edges of a fluorinated glass slide and a modified glass slide is clamped on top of it. The polymerization mixture is injected in between the mold and irradiated for 15 min at 12 mW/cm² with a 260 nm UV light. The mold is then carefully opened using a scalpel.

An inert, fluorinated glass slide is used as the bottom plate. The inability of covalent attachment of the growing polymer to the fluorinated glass slide allows for the whole polymer to stick to the top, modified glass slide during the separation process. The fluorinated glass slide can be reused several times. The resulting nonporous superficial layer can be easily removed by applying and rapidly removing adhesive film (Tesa tape) immediately after separating the plates while the layer is still wetted with porogen. A homogeneous, porous surface is formed. The plate is washed extensively with methanol and kept in methanol for several hours to remove unreacted monomers and porogens.

Polymerization mixture: 2-hydroxyethyl methacrylate (24 wt %), ethylene dimethacrylate (16 wt %), 1-decanol (12 wt %), cyclohexanol (48 wt %), and 2,2-dimethoxy-2-phenylacetophenone (1 wt % with respect to monomers).

Photografting: The polymer layer is wetted with the photografting mixture and covered with a fluorinated glass slide. A photomask is placed on top and it is irradiated for 30 min with 12 mW/cm² 260 nm UV light. The obtained pattern is washed extensively with methanol and stored in methanol for several hours to remove excess monomer and porogen, and for sterilization before cell culture. Photografting mixture: 2,2,3,3,3-pentafluoropropyl methacrylate (20 wt %), ethylene dimethacrylate (1.3 wt %), 1:3 (v/v) mixture of water:tert-butanol (78 wt %), benzophenone as the initiator (0.33 wt %).

### Materials and methods.

Krytox® GPL 103 was purchased from H. Costenoble GmbH (Germany). Tetrazolium salt 5-cyano-2,3-ditolyl tetrazolium chloride (CTC) was purchased from Polysciences, Inc. (Germany). 4',6-diamidino-2-phenylindole (DAPI) was purchased from Merck KGaA (Germany). All other chemicals were purchased from Sigma-Aldrich (Germany). The glass plates were Nexterion B glass from Schott (Germany). SEM images were obtained using the LEO 1530 Gemini scanning electron microscope (Zeiss, Germany). The samples were sputtered with a thin gold layer using a Cressington 108 auto sputter coater before SEM measurement. A UK 1115 digital camera from EHD imaging (Germany) was used to take images of the water droplet on the surface under ambient conditions. Image J software with a Dropsnake plugin was used to measure the water contact angle.

### Lubricant coating of porous polymer surfaces.

After cleaning and drying the polymer surface, an excess amount of Krytox® GPL 103 was applied to the surface. The lubricant coated surface was tilted for 2 hours to remove the excess lubricant layer before use.

### Stability test of the lubricant coated surface.

Stability of the lubricant coated surface was tested by measuring the water contact angles of the surfaces. Freshly prepared lubricant coated surfaces, lubricant coated surfaces after keeping 7 days in air and lubricant coated surfaces after keeping in BM2 medium flow (0.94ml/min) in a CDC bioreactor (BioSurface Technologies, Bozeman, USA) were measured.

### Bacterial strain and culture conditions.

*Pseudomonas fluorescencs* Pf-05 (ATCC BAA-477) and *Pseudomonas aeruginosa* environmental isolate overnight liquid cultures were grown at 30 °C and 37 °C respectively in BM2 minimal medium consisting of 62 mM potassium phosphate buffer (pH 7), 7 mM (NH₄)₂SO₄, 2 mM MgSO₄, 10 µM FeSO₄ and 0.4 % (w/v) glucose. The overnight cultures were diluted 1:10 with BM2 minimal medium to analyze the impact of Krytox® GPL 103 on bacterial growth (toxicity test) and the biofilm formation on different surfaces.

### Krytox® GPL 103 toxicity test.

The impact of Krytox® GPL 103 on bacterial suspensions was analyzed in a microtiter plate. A 2-fold serial dilution of Krytox® GPL 103 with sterile Milli-Q water (final volume 50 µl) was prepared in triplicate and 50 µl bacterial suspension (OD₆₀₀ nm 0.01) were added to each well. The microtiter plate was incubated at 30 °C on a shaker at 80 rpm. The optical density was measured after 1, 4, 8 and 22 hours at 560 nm (Labsystems Multiskan MS, Thermo Fisher Scientific, Schwerte, Germany).

### Adhesion assay.

The incubation of Krytox® GPL 103 coated porous surfaces with bacterial suspension was performed in a biofilm Plug Flow Reactor (PFR) with an in-house construction: length 29.0 cm, inner diameter 4.6 cm. For all experiments uncoated glass slides were used as reference. The biofilm reactor was inoculated with the diluted bacterial suspension. After a static 1 h incubation period, a constant flow rate of 0.94 ml/min was adjusted. The system was operated at room temperature (20 to 24 °C) for seven days. The experiment was repeated three times.

### Fluorescence staining and quantification of the bacterial adhesion.

The vital staining of bacteria is based on the intracellular enzymatic reduction of CTC to red fluorescent, water-insoluble formazan crystals. For the staining of the total cell count, a DNA specific DAPI was used. For maximum detection of respiring bacteria, the CTC solution was freshly prepared by adding CTC to BM2 minimal medium to a final concentration of 3.8 mM. The surfaces were removed from the reactor and gently washed with sterile cell wash buffer (5 mM Magnesium acetate, 10 mM Tris, pH 8.0). The different surfaces were incubated in the CTC staining solution with gently shaking in darkness at room temperature (20 to 24 °C) for 3 hours. Subsequently, the DAPI stain was added to a final concentration of 11.4 µM and incubated for 10 minutes. The surfaces were washed again with sterile cell wash buffer.

The bacterial adhesion on the surfaces were analyzed by epifluorescence microscopy with 200-fold magnification using the Axioplan 2 imaging system (Carl Zeiss, Oberkochen, Germany) with the filter sets for CTC (BP 546/12, FT 580, LP 590) and DAPI (G 365, FT 395, BP 445/50). Digital images of each sample were obtained with a Zeiss AxioCam MRm camera and the AxioVision 4.6 software. The surface coverage of the respiring bacteria (CTC stained, red) of five independent images per sample was determined with the BioFlux 200 software (Version 2.3.0.2; Fluxion Biosciences/ IUL Instruments GmbH, Königswinter, Germany).

### Example 1:

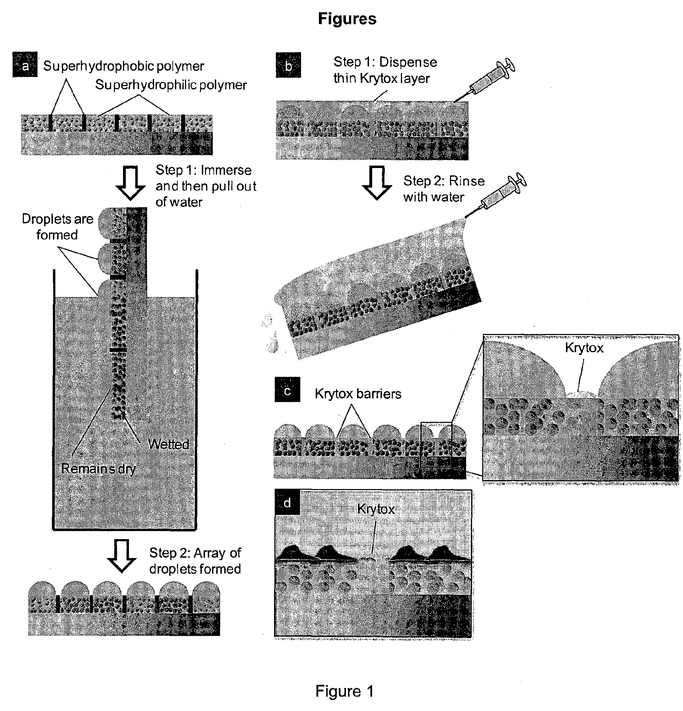

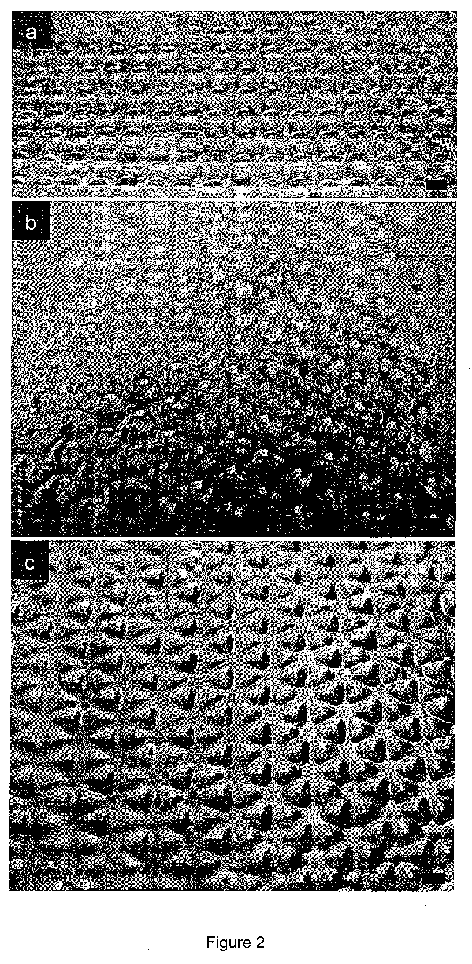

To create hydrophobic liquid-like regions to selectively pattern cells on a surface, the superhydrophobic regions were filled with a hydrophobic liquid before cell seeding (Figure 1). First, a nanoporous polymer surface with superhydrophilic spots on a superhydrophobic background was wetted with water to form droplets in the superhydrophilic spots. Second, a thin layer of hydrophobic liquid was spread over the surface while the water droplets were still contained in the superhydrophilic squares. Thus, the hydrophobic liquid only penetrated the superhydrophobic regions. Third, the hydrophobic liquid layer was washed under a stream of running water to remove the hydrophobic liquid layer from the superhydrophilic spots. The hydrophobic liquid layer that fills the nanoporous, superhydrophobic polymer does not easily wash away and thus remains stable. To pattern cells, the substrate is then immersed in medium containing cells and the water droplets in the superhydrophilic squares are replaced with medium containing cells. The cells preferentially adhere and grow in the superhydrophilic spots as opposed to the hydrophobic liquid background. This method eliminates the need for physical barriers to separate aqueous solutions and cells in different spots, and instead uses a liquid barrier. The hydrophobic liquid used in these experiments was the Krytox fluorinated oil developed by DuPont, a low molecular weight fluorine end-capped homopolymer of hexafluoropropylene epoxide. Figure 2 shows water droplets separated by Krytox barriers for various pattern geometries. The superhydrophobic barriers become transparent as soon as they are filled with the hydrophobic liquid. Herein, we will refer to superhydrophilic-hydrophobic liquid arrays as LiquidArrays.

### Example 2:

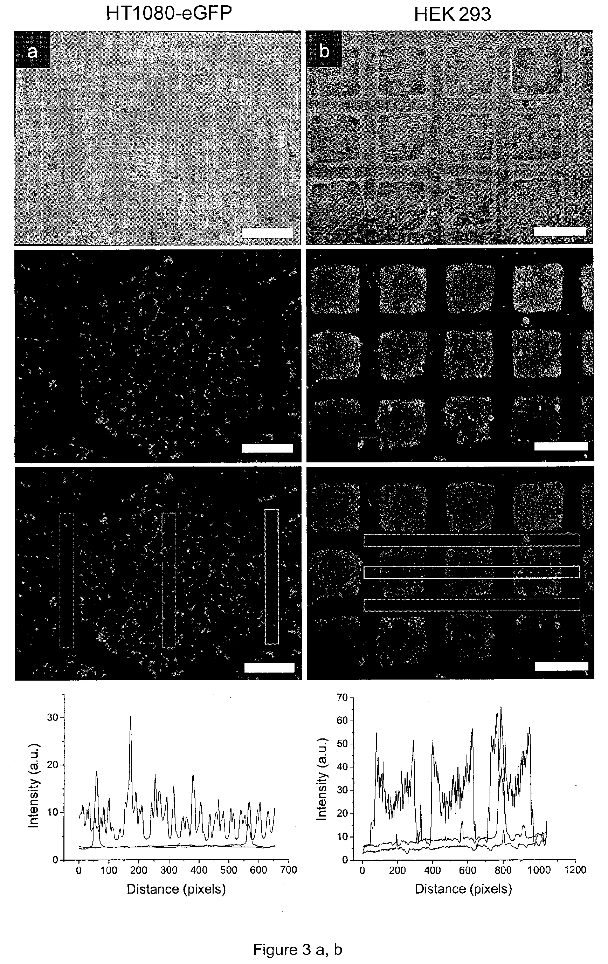

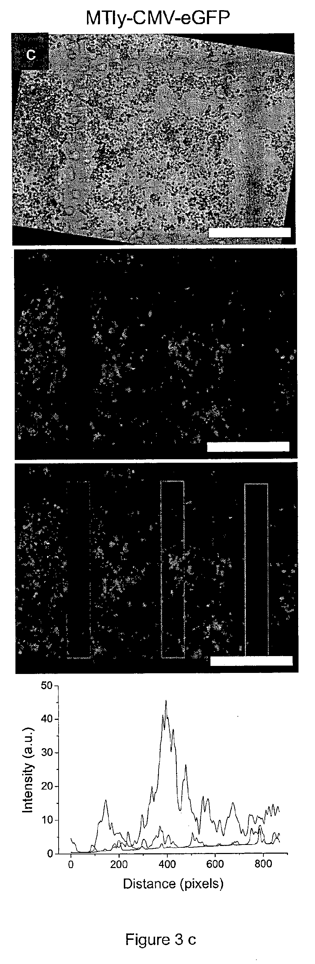

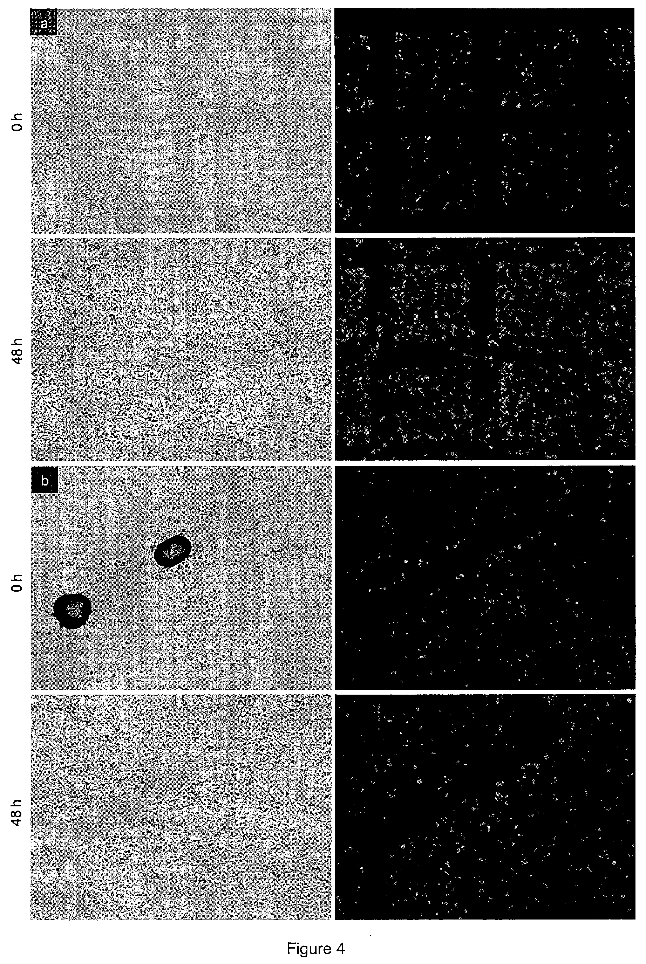

To demonstrate the cell-repellency of the hydrophobic liquid barriers, human fibrosarcoma cells stably expressing eGFP (HT1080-eGFP) and human embryonic kidney cells (HEK 293) were cultured on LiquidArrays for 3 days (Figure 3). A difference in the number of cells growing on the superhydrophilic spot compared to the hydrophobic liquid barrier is clearly observed. The cells that initially settle on a superhydrophilic spot migrate and proliferate within the superhydrophilic spot, and the hydrophobic liquid barriers are able to contain the cells within this superhydrophilic spot even when becoming very confluent. Many cells that initially settle on the hydrophobic liquid barrier are quick to migrate to a superhydrophilic region. The few cells that do remain on the hydrophobic liquid barrier after 3 days in culture have a rounder morphology compared to cells on the superhydrophilic region. This suggests that the cells do not readily adhere and grow on the hydrophobic liquid. However, this behavior seems to be dependent on the cell type and cell confluency. HT1080-eGFP cells were seeded at the same density on two different patterns: a square pattern with a smaller proportion of superhydrophilic area and a hexagonal pattern with a larger proportion of superhydrophilic area. When comparing the proportion of cells on the superhydrophilic regions versus the hydrophobic liquid barriers, the hexagonal LiquidArray was able to contain the HT1080-eGFP cells within the superhydrophilic spots better than the smaller square LiquidArray and there were fewer cells on the hydrophobic liquid barriers (Figure 4). This demonstrates that certain cell types, such as HT1080 cells, which are highly migrative and have a long, spindle morphology require more superhydrophilic area to grow and possibly wider hydrophobic liquid barriers to be confined within one superhydrophilic spot.

### Example 3:

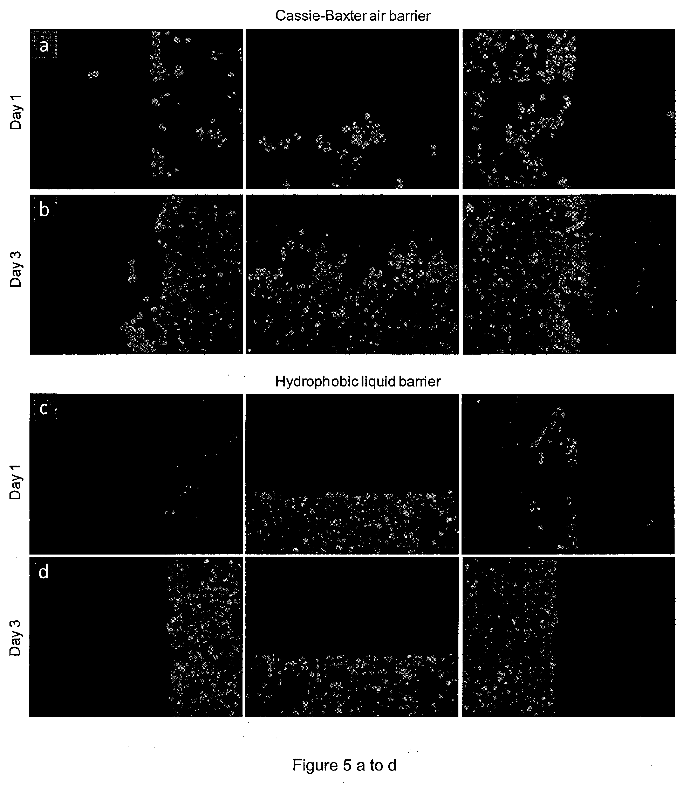

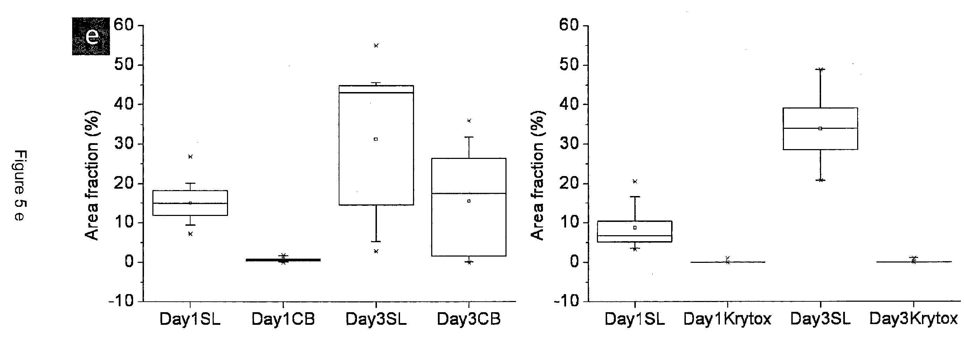

To prove the increased stability and cell-repellency of the hydrophobic liquid barriers compared to previously reported Cassie-Baxter air barriers, a single superhydrophilic square of 3 mm side length and 100 µm barrier width was filled with a 10 µl droplet of a cell suspension containing HEK 293 cells on both Cassie-Baxter air arrays and LiquidArrays. The cells were allowed to adhere overnight, the droplet was aspirated to remove unadhered cells, the substrate was immersed in cell culture medium, and the cells were cultured for 3 days. For both the Cassie-Baxter air and hydrophobic liquid arrays, the cells were mostly confined within the initially seeded superhydrophilic square (Figure 5A, C). However, after 3 days of culture there was a clear difference between the two barrier types (Figure 5B, D). The hydrophobic liquid barriers were much better at confining the cells within the superhydrophilic square and no cells were seen on the hydrophobic liquid barrier. The cells were clearly able to migrate across the Cassie-Baxter air barrier after 3 days in culture. This difference could be quantified and a 99.4% decrease in the number of cells on the hydrophobic liquid barriers compared to the Cassie-Baxter air barriers was calculated (Figure 5E).

### Example 4:

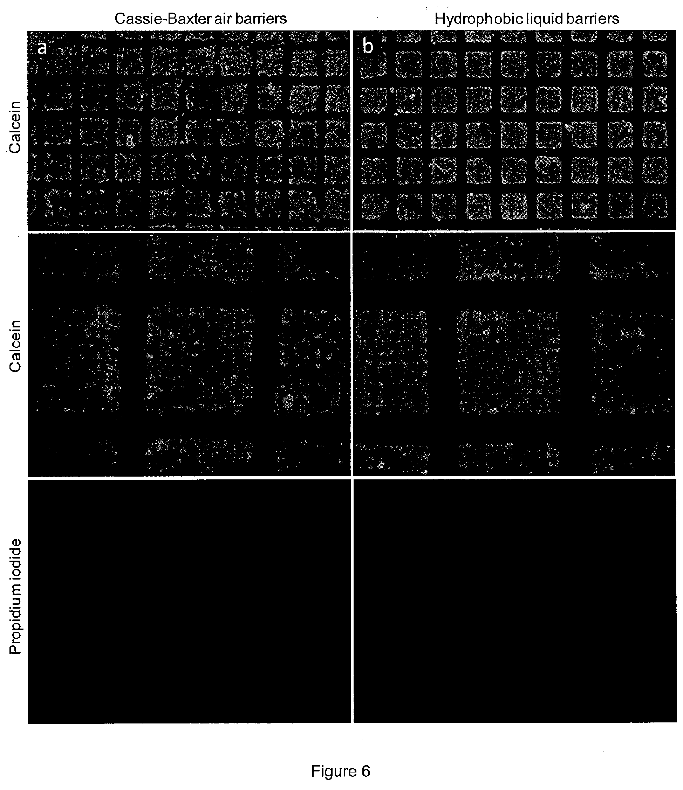

To determine whether cells on the hydrophobic liquid barriers were still viable although round in morphology, cell toxicity was tested. HEK 293 cells were cultured on LiquidArrays for 3 days and then stained with Calcein, Hoechst 33342, and propidium iodide (PI). Calcein and PI are used to indicate live and late apoptotic cells, respectively. As expected, there was low cell toxicity on both the LiquidArrays and Cassie-Baxter air arrays (Figure 6). However, cells on the hydrophobic liquid barriers are not firmly adhered so some cells may wash away during the staining procedure.

### Example 5:

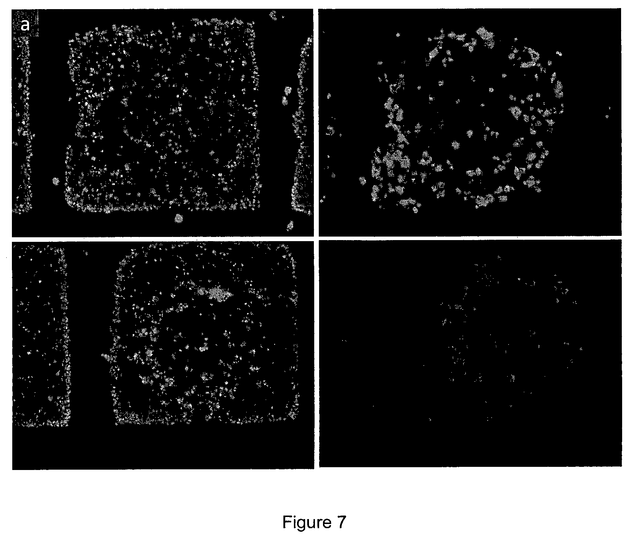

To demonstrate the application of LiquidArrays as cell microarrays, reverse cell transfection experiments were performed. First, droplets of transfection mixtures containing pCS2+-GFP and pCS2+-mCherry plasmid DNA were pipetted in a checkerboard pattern on the LiquidArray. The droplets were allowed to dry before forming the LiquidArray as described in Figure 1. Then, HEK 293 cells were seeded on the LiquidArrays and cultured for 48 h. The cell transfection efficiency was approximately 67% and there was no mixing of transfected cells between the neighboring superhydrophilic spots (Figure 7). Therefore, LiquidArrays can be used for cell patterning as well as microarray screening applications.

### Example 6:

A porous BMA-EDMA polymer surface with a thickness of ∼46µm was prepared on a glass substrate. The prepared surface showed a highly porous, globular structure. The porosity of the surface, determined by the content of porogens in the polymerization mixture, was 50%. The average globule size derived from surface SEM images was 1143±169 nm. The prepared porous BMA-EDMA surface, with a static water contact angle of ∼139±2°, was highly hydrophobic because of the hydrophobic nature of the polymer globules and the micro/nanoscale roughness of the surface. Krytox® GPL 103 lubricant was applied to the surface, upon which the liquid easily infiltrated the hydrophobic porous polymer film forming a thin lubricating film on top of the porous polymer. The pores of the porous surface became filled with the lubricant and a thin lubricant layer was fixed on the surface after the lubricant coating. The static water contact angle of the surface decreased from ∼139° to ∼116° after lubricant coating and the lubricant coated surface also showed a much lower water contact angle hysteresis (∼7°). These changes in water contact angles indicated that a smooth lubricant layer was formed on top of the porous surface after coating. The thickness of the lubricant layer on top of the microporous BMA-EDMA surface was about 13.1 µm estimated from a weight experiment.

### Example 7:

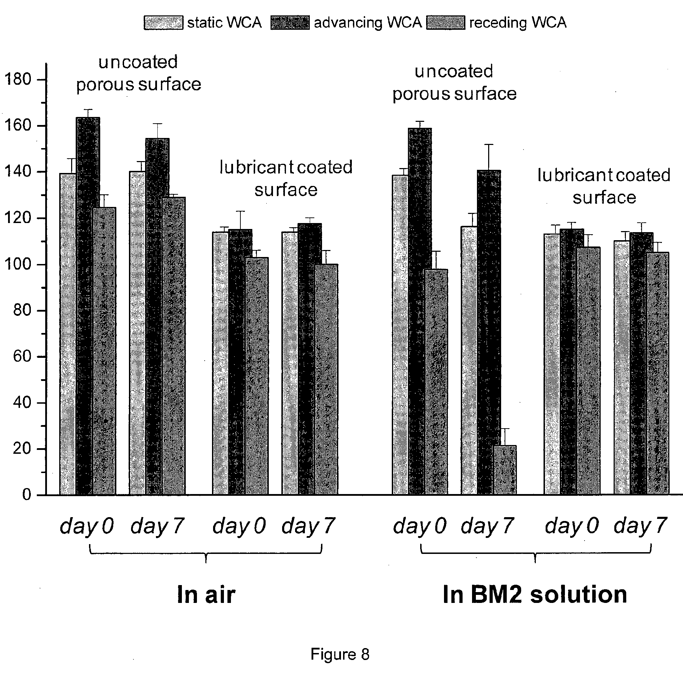

The stability of the lubricant coated surface is critical for the application of the surfaces. Mechanical damage and contamination to the surface usually decreases the receding water contact angle and thus increases the water contact angle hysteresis. Therefore, the stability of the lubricant coated surface both in air and in aqueous condition were tested by measuring the water contact angles. The water contact angles of the porous surfaces and the lubricant coated surfaces remained almost unchanged after keeping in air for 7 days, indicating that both uncoated surface and lubricant coated surface are quite stable in air. The porous surfaces and the lubricant coated surfaces were fixed horizontally in a CDC biofilm reactor and a 0.94mL/min BM2 minimal medium flow was applied (corresponding to a shear force of 0.0057 N/m²) inside the chamber. After keeping the surfaces for 7 days in the biofilm reactor, the water contact angles of the porous surfaces changed greatly. The static water contact angle of the porous surface decreased by ∼10° and the water contact angle hysteresis increased significantly. It is obvious that the sheer force generated by the BM2 minimal medium flow deteriorated the uncoated surface and therefore the water contact angle hysteresis of the uncoated surfaces increased greatly. The lubricant coated surface showed surprising mechanical stability under flow condition. All the static, advancing and receding water contact angles of the lubricant coated surface changed little after 7 days keeping in BM2 minimal medium flow in the CDC biofilm reactor (Figure 8).

### Example 8:

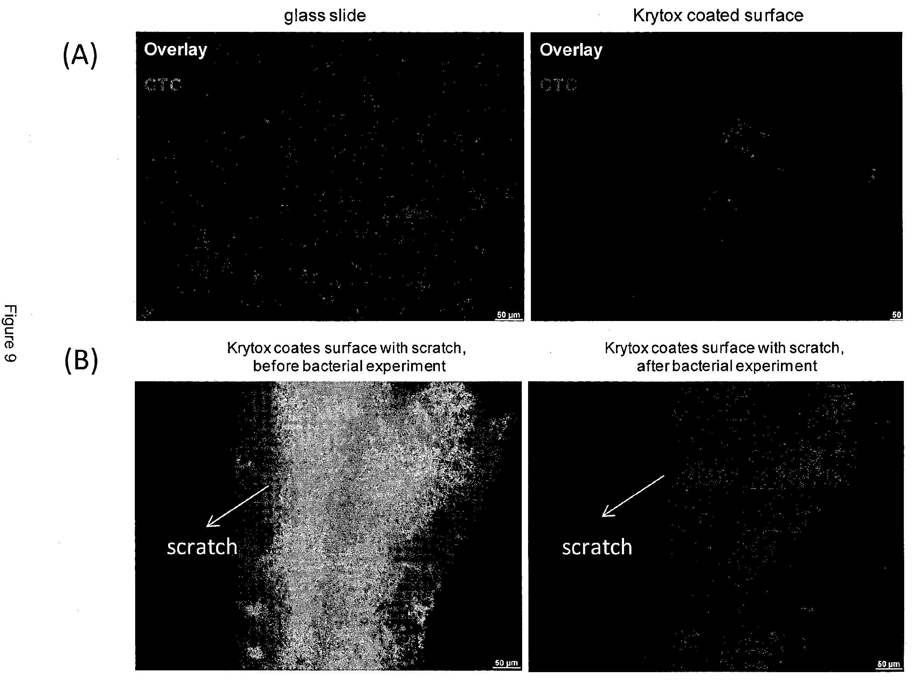

Seven day bacterial experiments were performed in a PFR reactor to check the antibacterial properties of the lubricant coated surface at a BM 2 medium flow rate of 0.94 mLmin⁻¹. Biofilm growth on the lubricant coated surfaces was greatly inhibited compared to the uncoated surfaces (Figure 9). The microscope images were quantified by measuring the surface area covered by biofilm in the microscope images. There is only average biofilm coverage of 4.5±6.4% on the lubricant coated surface, while the biofilm coverage is 51.7±32.8 % on a glass slide after 7 days bacterial experiments. The lubricant coated surface showed a significant lower biofilm surface coverage in comparison with the glass slide. Even after 14 days, there was only little biofilm formation on the lubricant coated surface.
The Krytox® GPL 103 toxicity test showed that lubricant itself does not have an antimicrobial effect. In BM2 minimal medium with up to 12.5 % Krytox the bacteria showed the same growth kinetics as in pure BM2 minimal medium. After 22 hours in all samples an OD_{560 nm} of 0.4 was reached.

The lubricant coated surface also showed excellent "self-healing" properties. Once physical damage happens on the lubricant surface, the lubricant will flow toward the damaged area by a surface energy driven capillary action and spontaneously refills the physical voids. The self healing properties of the lubricant coated surface were also tested. A scratch was made on the lubricant coated surface and then the scratched surface was used as surface for the 7 day bacterial experiment. It was found that there is almost no biofilm formation in the place where the surface is scratched after the 7 days bacterial experiment (Figure 9), indicating that the surface showed good "self healing" properties against physical damages like scratches.

In summary, the present results indicate that the surfaces can inhibit *Pseudomonas aeruginosa* biofilm formation in the long term. Due to the good stability and the excellent biofilm inhibition properties of the lubricant coated surface, the surfaces are promising for a number of antifouling applications.

## Claims

1. A substrate with a solid support coated with a porous hydrophobic polymer layer or film, wherein said polymer layer or film is coated with a water-immiscible hydrophobic liquid forming a stable film on the polymer surface; wherein the porous hydrophobic polymer layer or film comprises
(i) hydrophilic areas having a desired shape and size; and
(ii) hydrophobic areas having a desired shape and size;
wherein the hydrophilic areas are occupied by an aqueous solution and only the hydrophobic areas are occupied by the water-immiscible hydrophobic liquid; wherein the water-immiscible hydrophobic liquid is selected from the group consisting of perfluorinated lubricants, perfluorinated hydrocarbons, and organosilicone compounds;
wherein the hydrophilic areas are surrounded by the hydrophobic areas and are separated into a desired spatial pattern; and
wherein the porous hydrophobic polymer layer or film comprises a crosslinked polyvinyl monomer and a monovinyl monomer that have been copolymerized in the presence of an inert porogen.

2. A method of forming a protein-, cell- and bacteria-repellent surface, comprising the steps of:
(a) providing a substrate with a solid support coated with a porous hydrophobic polymer layer or film; and
(b) applying a water-immiscible hydrophobic liquid to said porous hydrophobic polymer layer or film;
wherein the porous hydrophobic polymer layer or film comprises
(i) hydrophilic areas having a desired shape and size; and
(ii) hydrophobic areas having a desired shape and size;
wherein an aqueous solution is applied to said porous polymer layer or film after step (a) and before step (b), so that said hydrophilic areas are occupied by said aqueous solution and only the hydrophobic areas are occupied by the water-immiscible hydrophobic liquid;
wherein the water-immiscible hydrophobic liquid is selected from the group consisting of perfluorinated lubricants, perfluorinated hydrocarbons, and organosilicone compounds;
wherein the hydrophilic areas are surrounded by the hydrophobic areas and are separated into a desired spatial pattern; and
wherein the porous hydrophobic polymer layer or film comprises a crosslinked polyvinyl monomer and a monovinyl monomer that have been copolymerized in the presence of an inert porogen.

3. A method of preventing or retarding the formation of biofilms, adhesion of cells and bacteria, and/or migration of cells and bacteria on a surface or on parts of a surface, comprising the steps of:
(a) providing on said surface or parts of said surface a porous hydrophobic polymer layer or film; and
(b) applying a water-immiscible hydrophobic liquid to said porous hydrophobic polymer layer or film;
wherein the porous hydrophobic polymer layer or film comprises
(i) hydrophilic areas having a desired shape and size; and
(ii) hydrophobic areas having a desired shape and size;
wherein the hydrophilic areas are occupied by an aqueous solution and only the hydrophobic areas are occupied by the water-immiscible hydrophobic liquid;
wherein the water-immiscible hydrophobic liquid is selected from the group consisting of perfluorinated lubricants, perfluorinated hydrocarbons, and organosilicone compounds;
wherein the hydrophilic areas are surrounded by the hydrophobic areas and are separated into a desired spatial pattern; and
wherein the porous hydrophobic polymer layer or film comprises a crosslinked polyvinyl monomer and a monovinyl monomer that have been copolymerized in the presence of an inert porogen.

4. Use of a porous hydrophobic polymer layer or film that is coated with a water-immiscible hydrophobic liquid for forming a protein-, cell- and bacteria-repellent surface;
wherein the porous hydrophobic polymer layer or film comprises
(i) hydrophilic areas having a desired shape and size; and
(ii) hydrophobic areas having a desired shape and size;
wherein the hydrophilic areas are occupied by an aqueous solution and only the hydrophobic areas are occupied by the water-immiscible hydrophobic liquid;
wherein the water-immiscible hydrophobic liquid is selected from the group consisting of perfluorinated lubricants, perfluorinated hydrocarbons, and organosilicone compounds;
wherein the hydrophilic areas are surrounded by the hydrophobic areas and are separated into a desired spatial pattern; and
wherein the porous hydrophobic polymer layer or film comprises a crosslinked polyvinyl monomer and a monovinyl monomer that have been copolymerized in the presence of an inert porogen.

5. The substrate of claim 1, wherein the perfluorinated lubricant is a perfluoropolyether.

6. The methods of claim 2 or claim 3, wherein the perfluorinated lubricant is a perfluoropolyether.

7. The use of claim 4, wherein the perfluorinated lubricant is a perfluoropolyether.

## Patentansprüche

1. Substrat mit einem festen Träger, beschichtet mit einer/m porösen hydrophoben Polymerschicht oder -film, wobei die/der Polymerschicht oder -film mit einer mit Wasser nicht mischbaren hydrophoben Flüssigkeit beschichtet ist, die einen stabilen Film auf der Polymeroberfläche bildet;
wobei die/der poröse hydrophobe Polymerschicht oder -film umfasst
(i) hydrophile Flächen mit einer gewünschten Form und Größe; und
(ii) hydrophobe Flächen mit einer gewünschten Form und Größe;
wobei die hydrophilen Flächen von einer wässrigen Lösung besetzt sind und nur die hydrophoben Flächen von der mit Wasser nicht mischbaren hydrophoben Flüssigkeit besetzt sind;
wobei die mit Wasser nicht mischbare hydrophobe Flüssigkeit aus der Gruppe, bestehend aus perfluorierten Schmiermitteln, perfluorierten Kohlenwasserstoffen und Organosiliciumverbindungen, ausgewählt ist;
wobei die hydrophilen Flächen von den hydrophoben Flächen umgeben sind und in einem gewünschten räumlichen Muster aufgeteilt sind; und
wobei die/der poröse hydrophobe Polymerschicht oder -film ein vernetztes Polyvinylmonomer und ein Monovinylmonomer, die in der Gegenwart eines inerten Porenbildners copolymerisiert wurden, umfasst.

2. Verfahren des Bildens einer protein-, zell- und bakterienabweisenden Oberfläche, umfassend die Schritte:
(a) Bereitstellen eines Substrats mit einem festen Träger, beschichtet mit einer/m porösen hydrophoben Polymerschicht oder -film; und
(b) Auftragen einer mit Wasser nicht mischbaren hydrophoben Flüssigkeit auf die/den poröse/n hydrophobe/n Polymerschicht oder -film;
wobei die/der poröse hydrophobe Polymerschicht oder -film umfasst
(i) hydrophile Flächen mit einer gewünschten Form und Größe; und
(ii) hydrophobe Flächen mit einer gewünschten Form und Größe;
wobei eine wässrige Lösung auf die/den poröse/n Polymerschicht oder -film nach Schritt (a) und vor Schritt (b) aufgetragen wird, so dass die hydrophilen Flächen von der wässrigen Lösung besetzt sind und nur die hydrophoben Flächen von der mit Wasser nicht mischbaren hydrophoben Flüssigkeit besetzt sind;
wobei die mit Wasser nicht mischbare hydrophobe Flüssigkeit aus der Gruppe, bestehend aus perfluorierten Schmiermitteln, perfluorierten Kohlenwasserstoffen und Organosiliciumverbindungen, ausgewählt ist;
wobei die hydrophilen Flächen von den hydrophoben Flächen umgeben sind und in einem gewünschten räumlichen Muster aufgeteilt sind; und
wobei die/der poröse hydrophobe Polymerschicht oder -film ein vernetztes Polyvinylmonomer und ein Monovinylmonomer, die in der Gegenwart eines inerten Porenbildners copolymerisiert wurden, umfasst.

3. Verfahren des Verhinderns oder Verlangsamens der Bildung von Biofilmen, Anhaftung von Zellen und Bakterien und/oder Migration von Zellen und Bakterien auf einer Oberfläche oder auf Teilen einer Oberfläche, umfassend die Schritte:
(a) Bereitstellen einer/s porösen hydrophoben Polymerschicht oder -films auf der Oberfläche oder Teilen der Oberfläche; und
(b) Auftragen einer mit Wasser nicht mischbaren hydrophoben Flüssigkeit auf die/den poröse/n hydrophobe/n Polymerschicht oder -film;
wobei die/der poröse hydrophobe Polymerschicht oder -film umfasst
(i) hydrophile Flächen mit einer gewünschten Form und Größe; und
(ii) hydrophobe Flächen mit einer gewünschten Form und Größe;
wobei die hydrophilen Flächen von einer wässrigen Lösung besetzt sind und nur die hydrophoben Flächen von der mit Wasser nicht mischbaren hydrophoben Flüssigkeit besetzt sind;
wobei die mit Wasser nicht mischbare hydrophobe Flüssigkeit aus der Gruppe, bestehend aus perfluorierten Schmiermitteln, perfluorierten Kohlenwasserstoffen und Organosiliciumverbindungen, ausgewählt ist;
wobei die hydrophilen Flächen von den hydrophoben Flächen umgeben sind und in einem gewünschten räumlichen Muster aufgeteilt sind; und
wobei die/der poröse hydrophobe Polymerschicht oder -film ein vernetztes Polyvinylmonomer und ein Monovinylmonomer, die in der Gegenwart eines inerten Porenbildners copolymerisiert wurden, umfasst.

4. Verwendung einer/s porösen hydrophoben Polymerschicht oder -films, die/der mit einer mit Wasser nicht mischbaren hydrophoben Flüssigkeit für das Bilden einer protein-, zell- und bakterienabweisenden Oberfläche beschichtet ist; wobei die/der poröse hydrophobe Polymerschicht oder -film umfasst
(i) hydrophile Flächen mit einer gewünschten Form und Größe; und
(ii) hydrophobe Flächen mit einer gewünschten Form und Größe;
wobei die hydrophilen Flächen von einer wässrigen Lösung besetzt sind und nur die hydrophoben Flächen von der mit Wasser nicht mischbaren hydrophoben Flüssigkeit besetzt sind;
wobei die mit Wasser nicht mischbare hydrophobe Flüssigkeit aus der Gruppe, bestehend aus perfluorierten Schmiermitteln, perfluorierten Kohlenwasserstoffen und Organosiliciumverbindungen, ausgewählt ist;
wobei die hydrophilen Flächen von den hydrophoben Flächen umgeben sind und in einem gewünschten räumlichen Muster aufgeteilt sind; und
wobei die/der poröse hydrophobe Polymerschicht oder -film ein vernetztes Polyvinylmonomer und ein Monovinylmonomer, die in der Gegenwart eines inerten Porenbildners copolymerisiert wurden, umfasst.

5. Substrat nach Anspruch 1, wobei das perfluorierte Schmiermittel ein Perfluorpolyether ist.

6. Verfahren nach Anspruch 2 oder Anspruch 3, wobei das perfluorierte Schmiermittel ein Perfluorpolyether ist.

7. Verwendung nach Anspruch 4, wobei das perfluorierte Schmiermittel ein Perfluorpolyether ist.

## Revendications

1. Substrat comportant un support solide revêtu d'un film ou couche polymère hydrophobe poreux, dans lequel ledit film ou couche polymère est revêtu d'un liquide hydrophobe non miscible dans l'eau formant un film stable sur la surface polymère ;
dans lequel le film ou couche polymère hydrophobe poreux comprend :
(i) des zones hydrophiles présentant des forme et taille désirées ; et
(ii) des zones hydrophobes présentant des forme et taille désirées ;
dans lequel les zones hydrophiles sont occupées par une solution aqueuse et seules les zones hydrophobes sont occupées par le liquide hydrophobe non miscible dans l'eau ;
dans lequel le liquide hydrophobe non miscible dans l'eau est sélectionné à partir du groupe constitué par des lubrifiants perfluorés, des hydrocarbures perfluorés ; et des composés organosilicones ;
dans lequel les zones hydrophiles sont entourées par les zones hydrophobes et sont séparées suivant un motif spatial désiré ; et
dans lequel le film ou couche polymère hydrophobe poreux comprend un monomère polyvinyle réticulé et un monomère monovinyle qui ont été copolymérisés en la présence d'un porogène inerte.

2. Procédé de formation d'une surface répulsive de protéines, cellules et bactéries, comprenant les étapes de :
(a) préparation d'un substrat avec un support solide revêtu d'un film ou couche polymère hydrophobe poreux ; et
(b) application d'un liquide hydrophobe non miscible dans l'eau sur ledit film ou couche polymère hydrophobe poreux ;
dans lequel le film ou couche polymère hydrophobe poreux comprend :
(i) des zones hydrophiles présentant des forme et taille désirées ; et
(ii) des zones hydrophobes présentant des forme et taille désirées ;
dans lequel une solution aqueuse est appliquée sur ledit film ou couche polymère poreux après l'étape (a) et avant l'étape (b), de telle sorte que lesdites zones hydrophiles sont occupées par ladite solution aqueuse et seules les zones hydrophobes sont occupées par le liquide hydrophobe non miscible dans l'eau ;
dans lequel le liquide hydrophobe non miscible dans l'eau est sélectionné à partir du groupe constitué par des lubrifiants perfluorés, des hydrocarbures perfluorés et des composés organosilicones ;
dans lequel les zones hydrophiles sont entourées par les zones hydrophobes et sont séparées suivant un motif spatial désiré ; et
dans lequel le film ou couche polymère hydrophobe poreux comprend un monomère polyvinyle réticulé et un monomère monovinyle qui ont été copolymérisés en la présence d'un porogène inerte.

3. Procédé destiné à empêcher ou retarder la formation de biofilms, l'adhérence de cellules et bactéries et/ou la migration de cellules et bactéries sur une surface ou sur des parties d'une surface, comprenant les étapes de :
(a) formation sur ladite surface ou des parties de ladite surface d'un film ou couche polymère hydrophobe poreux ; et
(b) application d'un liquide hydrophobe non miscible dans l'eau sur ledit film ou couche polymère hydrophobe poreux ;
dans lequel ledit film ou couche polymère hydrophobe poreux comprend :
(i) des zones hydrophiles présentant des forme et taille désirées ; et
(ii) des zones hydrophobes présentant des forme et taille désirées ;
dans lequel les zones hydrophiles sont occupées par une solution aqueuse et seules les zones hydrophobes sont occupées par le liquide hydrophobe non miscible dans l'eau ;
dans lequel le liquide hydrophobe non miscible dans l'eau est sélectionné à partir du groupe constitué par des lubrifiants perfluorés, des hydrocarbures perfluorés et des composés organosilicones ;
dans lequel les zones hydrophiles sont entourées par les zones hydrophobes et sont séparées suivant un motif spatial désiré ; et
dans lequel le film ou couche polymère hydrophobe poreux comprend un monomère polyvinyle réticulé et un monomère monovinyle qui ont été copolymérisés en la présence d'un porogène inerte.

4. Utilisation d'un film ou couche polymère hydrophobe poreux qui est revêtu d'un liquide hydrophobe non miscible dans l'eau afin de former une surface répulsive de protéines, cellules et bactéries ;
dans laquelle le film ou couche polymère hydrophobe poreux comprend :
(i) des zones hydrophiles présentant des forme et taille désirées ; et
(ii) des zones hydrophobes présentant des forme et taille désirées ;
dans lequel les zones hydrophiles sont occupées par une solution aqueuse et seules les zones hydrophobes sont occupées par le liquide hydrophobe non miscible dans l'eau ;
dans lequel le liquide hydrophobe non miscible dans l'eau est sélectionné à partir du groupe constitué par des lubrifiants perfluorés, des hydrocarbures perfluorés et des composés organosilicones ;
dans lequel les zones hydrophiles sont entourées par les zones hydrophobes et sont séparées suivant un motif spatial désiré ; et
dans lequel le film ou couche polymère hydrophobe poreux comprend un monomère polyvinyle réticulé et un monomère monovinyle qui ont été copolymérisés en la présence d'un porogène inerte.

5. Substrat selon la revendication 1, dans lequel le lubrifiant perfluoré est un perfluoropolyéther.

6. Procédé selon la revendication 2 ou 3, dans lequel le lubrifiant perfluoré est un perfluoropolyéther.

7. Utilisation selon la revendication 4, dans laquelle le lubrifiant perfluoré est un perfluoropolyéther.
